# EUROPEAN PATENT SPECIFICATION

(11) **EP 2 297 574 B1**
(45) Date of publication and mention of the grant of the patent: **08.01.2014**
(21) Application number: 09794578.6
(22) Date of filing: 20.05.2009
(51) Int. Cl.: G01N 33/487, C12Q 1/00

(54) **BIO-SENSOR**
BIOSENSOR
BIOCAPTEUR

(30) Priority: 09.07.2008 KR 20080066615
(43) Date of publication of application: 23.03.2011
(73) Proprietor: All Medicus Co., Ltd, Dongan-gu Anyang-si Gyeonggi-do 431-716 (KR)
(72) Inventor: AHN, Yon Chan, Uiwang-si Gyeonggi-do 437-770 (KR); CHOI, Yong Bok, Gunpo-si Gyeonggi-do 435-050 (KR); PARK, Mi Suk, Uiwang-si Gyeonggi-do 437-770 (KR)
(74) Representative: von Kreisler Selting Werner
(86) International application number: PCT/KR2009/002660
(87) International publication number: WO 2010/005172

(56) References cited:
- EP-A1- 1 291 650
- EP-A1- 1 437 589
- EP-A1- 1 691 192
- EP-A2- 0 634 488
- WO-A1-2007/126920
- US-A1- 2004 067 166
- US-A1- 2007 205 114

## Description

### Technical Field

The present invention relates to a biosensor and, more particularly, to a biosensor, in which sensing electrodes are formed on an upper insulating substrate and a lower insulating substrate such that, using the sensing electrodes, a measuring device can determine whether a sample (e.g., blood) injected through a sample injection port is completely filled in a sample path and determine a time point at which the sample is injected and a time point at which the sample is completely filled. Therefore, it is possible to estimate the flow characteristics of the sample and the flow rate and use the estimated values to correct the measurement result, thus obtaining a more accurate measurement result.

### Background Art

A biosensor is a device that converts information on an analyte into a detectable signal such as a color, fluorescent, or electrical signal using a biological component or imitating the biological component.

Especially, since a biosensor that utilizes a biological enzyme has excellent sensitivity and reaction specificity, it is expected that it will be useful in a wide range of applications such as medical/pharmaceutical field, process measurement in bioindustry, environmental measurement, stability evaluation of chemical materials, etc.

Measurement of chemical components in vitro is medically important, and thus the biosensors are widely used in the analysis of biological samples such as blood in the medical diagnosis field.

Among them, a biosensor using an enzyme analysis method in which a specific reaction between an enzyme and a substrate or between an enzyme and an inhibitor is used has advantages of simple application, excellent detection sensitivity, and fast response, and thus it is most widely used in hospitals and clinical chemistry analysis.

The enzyme analysis method for the measurement of chemical components in vitro may be classified into a chromatographic method for measuring optical transmittance by a spectroscopic method before and after an enzyme reaction, and an electrochemical method for measuring an electrochemical signal.

Compared to the electrochemical method, the chromatographic method has difficulties in analyzing critical biomaterials because the measurement time is long, a large amount of same is required, and measurement errors occur due to turbidity in biological samples.

Therefore, the electrochemical method, in which an electrode system including a plurality of electrodes is formed on a plastic film (insulating substrate) by etching, screen printing, or sputtering, and a reagent is fixed onto the electrodes, such that a specific substance in a sample is quantitatively measured by applying a predetermined electric potential to the sample, has been widely applied to the biosensor using the enzyme analysis method.

In the case where the electrochemical method is employed, a measuring device for obtaining information on biological samples from the biosensor is required, and the measuring device includes a socket electrically connected to thin film electrodes of the biosensor. Accordingly, when the thin film electrodes of the biosensor are inserted into the measuring device through an inlet port, the thin film electrodes are connected to terminals formed in the socket of the measuring device such that the measuring device in an ON state obtains information on the biological material as an analyte.

One of the most widely used biosensors employing the electrochemical method is a blood glucose meter, with which everyone can measure the concentration of glucose (sugar) in blood by easily taking a drop of blood.

In the case of insulin-dependent diabetics who should measure blood glucose two to three times a day, they take a drop of blood by pricking a finger with a lancet to measure blood glucose. At this time, the blood glucose meter is used to measure blood glucose level from an electrical signal generated by an electrochemical reaction between a reactant in the biosensor and a sample (e.g., blood) taken from a diabetic.

The biosensor typically includes an electrode system having a plurality of electrodes formed on an insulating substrate by screen printing, and an enzyme reaction layer formed on the electrode system and including a hydrophilic polymer, an oxidoreductase and an electron acceptor. When a user injects a sample containing a substrate (glucose) through a sample injection port of the biosensor to come in contact with the enzyme reaction layer, the enzyme reaction layer dissolves the sample, the substrate in the sample reacts with an enzyme and is oxidized, and thus the electron acceptor is reduced. At this time, an oxidation current obtained when the reduced electron acceptor is electrochemically oxidized is measured by the measuring device, thereby obtaining the concentration of the substrate contained in the sample.

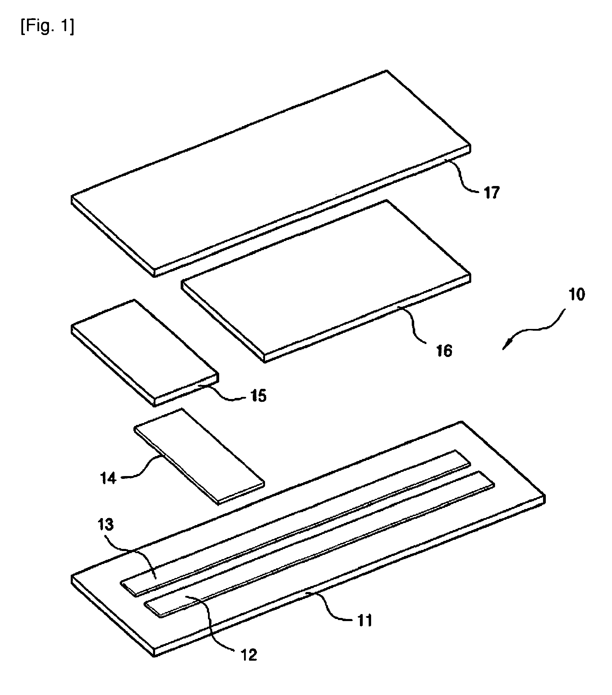

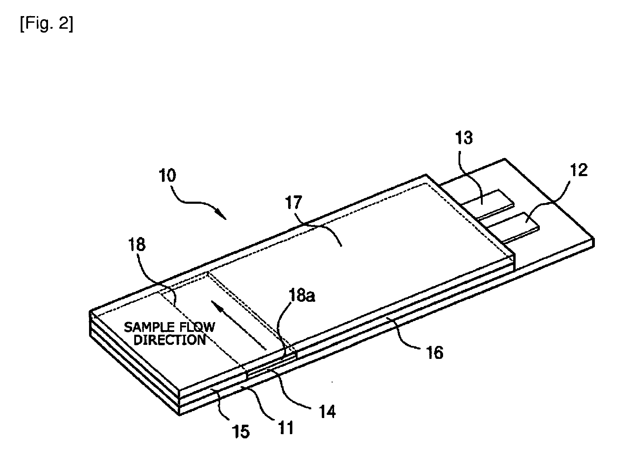

FIGS. 1 and 2 are diagrams showing a basic configuration of an electrochemical biosensor, and the configuration of a conventional biosensor will be described with reference to FIGS. 1 and 2. FIG. 1 is an exploded perspective view and FIG. 2 is an assembled perspective view.

As shown in FIGS. 1 and 2, a biosensor 10 includes a lower insulating substrate 11, on which a working electrode 12 and a reference electrode 13 are stacked in the longitudinal direction, and an enzyme reaction layer 14 as a reagent is immobilized onto the working electrode 12 and the reference electrode 13 in the width direction. The electrodes 12 and 13 are formed by a thin film formation process such as etching, screen printing, or sputtering.

Moreover, spacers 15 and 16 are stacked on the top of the lower insulating substrate 11 on which the electrodes 12 and 13 are formed such that a sample (e.g., blood) is appropriately introduced into the entire enzyme reaction layer 14. Then, an upper insulating substrate 17 is stacked on the top of the spacers 15 and 16 such that the upper and lower insulating substrates 17 and 11, spaced from each other by the spacers 15 and 16, form a sample path 18 having a capillary structure on the top of the enzyme reaction layer 14.

Here, the working electrode 12 and the reference electrode 13 are insulated from each other by the spacers 15 and 16, and an inlet of the sample path 18, formed by the upper and lower insulating substrates 17 and 11 and the spacers 15 and 16, corresponds to a sample injection port 18a through which the sample is injected. Moreover, the working electrode 12 and the reference electrode 13 are exposed at a connection terminal of the biosensor 10 such that they can be connected to terminals formed in a socket of a measuring device (not shown) when the biosensor 10 is inserted into the measuring device.

When the working electrode 12 and the reference electrode 13 are electrically connected to the measuring device in the above manner, an electrical signal generated by a reaction between a component (e.g., blood glucose) in the sample and the enzyme reaction layer 14 is detected. At this time, the oxidation-reduction reaction between the component in the sample and the enzyme reaction layer 14 takes place at the working electrode 12 to generate a predetermined current by an electrochemical mechanism at the working electrode 12. When the thus generated current is applied to the measuring device, the measuring device measures the current applied from the biosensor 10 to quantitatively analyze the component in the sample and display the result on a display.

Meanwhile, when blood is injected through the sample injection port 18a and introduced into the enzyme reaction layer 14, the component in blood reacts with the enzyme reaction layer 14 in the biosensor 10 to generate a current. At this time, it is necessary that a sufficient amount of blood be filled in the sample path 18 on the enzyme reaction layer 14 in order to obtain an accurate measurement result.

That is, the blood injected into the sample injection port 18a and present in the sample path 18 on the enzyme reaction layer 14 sequentially passes through the first electrode 12 and the second electrode 13. At this time, only if the sample path 18 is completely filled with the blood reaching the second electrode 13, an accurate measurement is possible.

However, since the reaction takes place immediately after the blood is in contact with the second electrode 13, the measurement may be made in a state that the blood has not yet been filled in the sample path 18 and, in this case, an error may occur. Accordingly, it is necessary to determine whether a sufficient amount of blood reaches the second electrode and is filled therein. In the case of the conventional biosensor, it is impossible to accurately determine the time point at which the blood starts coming in contact with the second electrode 13 and is filled in the sample path 18.

Moreover, the rate at which the blood flows in the sample path 18 may differ according to the flow characteristics of blood, and the flow characteristics of blood may affect the measurement result. Accordingly, during the analysis, it is possible to correct the measurement error according to the flow characteristics of blood with a difference in flow rate. Furthermore, it is possible to estimate the properties of the sample from the difference in flow rate.

However, in the case of the conventional biosensor, it is impossible to determine the rate at which the blood flows in the sample path 18, and thus it is impossible to correct the measurement error according to the flow characteristics of blood.

### Disclosure of Invention

### Technical Problem

Accordingly, the present invention has been made to solve the above-describe problems, and an object of the present invention is to provide a biosensor, in which sensing electrodes are formed on an upper insulating substrate and a lower insulating substrate such that, using the sensing electrodes, a measuring device can determine whether a sample (e.g., blood) injected through a sample injection port is completely filled in a sample path and determine a time point at which the sample is injected and a time point at which the sample is completely filled. Therefore, it is possible to estimate the flow characteristics of the sample and the flow rate and use the estimated values to correct the measurement result, thus obtaining a more accurate measurement result.

### Technical Solution

To accomplish the above objects of the present invention, there is provided a biosensor comprising an upper insulating substrate, a lower insulating substrate, a working electrode, a reference electrode, the working electrode and the reference electrode being formed on the inner surface of the lower insulating substrate, spacers interposed between the upper insulating substrate and the lower insulating substrate to form a sample path, and an enzyme reaction layer immobilized onto the working electrode and the reference electrode along the sample path in the width direction, wherein at least one sensing electrode is formed on the upper insulating substrate and the lower insulating substrate, respectively, the sensing electrodes being disposed around a sample injection port and at the opposite side of the sample injection port, respectively, with the working electrode and the reference electrode interposed therebetween, and extending from the sample path to a connection terminal to be connected to a socket of a measuring device.

In a first embodiment of the present invention, sensing electrodes are formed on the inner surface of the upper insulating substrate and the inner surface of the lower insulating substrate at positions opposite to each other around the sample injection port, respectively, and a sensing electrode is formed on the inner surface of the lower insulating substrate at the opposite side of the sample injection port.

In a second embodiment of the present invention, sensing electrodes are formed on the inner surface of the upper insulating substrate and the inner surface of the lower insulating substrate at positions opposite to each other around the sample injection port and at the opposite side of the sample injection port, respectively.

In a third embodiment of the present invention, a sensing electrode is formed on the inner surface of the upper insulating substrate at the opposite side of the sample injection port, and a sensing electrode is formed on the inner surface of the lower insulating substrate around the sample injection port.

In a fourth embodiment of the present invention, a sensing electrode is formed on the inner surface of the upper insulating substrate around the sample injection port, and a sensing electrode is formed on the inner surface of the lower insulating substrate at the opposite side of the sample injection port.

### Advantageous Effects

According to the biosensor of the present invention in which the sensing electrodes are formed on the upper insulating substrate and the lower insulating substrate such that, using the sensing electrodes, the measuring device can determine whether a sample (e.g., blood) is completely filled in the sample path when the sample is injected through the sample injection port, thus obtaining a more accurate measurement result.

Moreover, since the measuring device can determine a time point at which the sample is injected and a time point at which the sample is completely filled by means of the electrodes provided in the biosensor, it is possible to estimate the flow characteristics of the sample and the flow rate and use the estimated values to correct the measurement result, thus obtaining a more accurate measurement result.

### Brief Description of Drawings

FIGS. 1 and 2 are diagrams showing a basic configuration of an electrochemical biosensor;
FIGS. 3 and 4 are perspective views showing a biosensor in accordance with a first embodiment of the present invention;
FIG. 5 is an exploded perspective view showing a biosensor in accordance with a second embodiment of the present invention;
FIG. 6 is an exploded perspective view showing a biosensor in accordance with a third embodiment of the present invention; and
FIG. 7 is an exploded perspective view showing a biosensor in accordance with a fourth embodiment of the present invention.

### Best Mode for Carrying out the Invention

Hereinafter reference will now be made in detail to various embodiments of the present invention, examples of which are illustrated in the accompanying drawings and described below. While the invention will be described in conjunction with exemplary embodiments, it will be understood that present description is not intended to limit the invention to those exemplary embodiments. On the contrary, the invention is intended to cover not only the exemplary embodiments, but also various alternatives, modifications, equivalents and other embodiments, which may be included within the spirit and scope of the invention as defined by the appended claims.

The present invention aims at providing a biosensor in which sensing electrodes are formed on an upper insulating substrate and a lower insulating substrate to determine whether a sample (e.g., blood) injected through a sample injection port is completely filled in a sample path.

For this purpose, the present invention provides a biosensor, in which at least one sensing electrode is formed on an upper insulating substrate and a lower insulating substrate, respectively, the sensing electrodes being disposed around a sample injection port and at the opposite side of the sample injection port, respectively, with a working electrode and a reference electrode interposed therebetween, and extending from a sample path to a connection terminal to be connected to a socket of a measuring device.

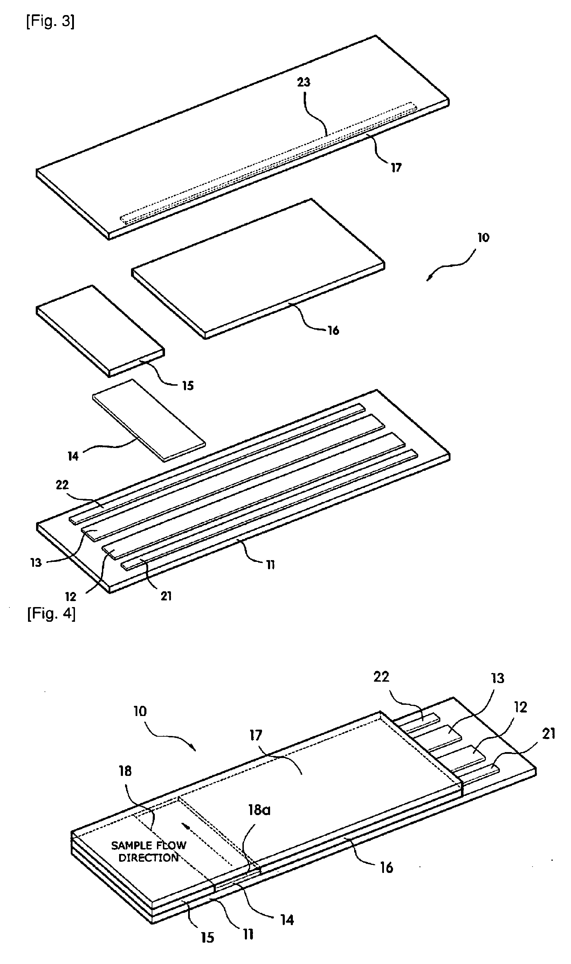

FIGS. 3 and 4 are perspective views showing a biosensor in accordance with a first embodiment of the present invention, in which FIG. 3 is an exploded perspective view and FIG. 4 is an assembled perspective view.

As shown in FIGS. 3 and 4, a biosensor 10 includes an upper insulating substrate 17, a lower insulating substrate 11, a working electrode 12, a reference electrode 13, spacers 15 and 16, and an enzyme reaction layer 14. The working electrode 12 and the reference electrode 13 are formed on an upper surface (inner surface) of the lower insulating substrate 11. The spacers 15 and 16 are interposed between the upper insulating substrate 17 and the lower insulating substrate 11 to form a sample path 18. The enzyme reaction layer 14 is immobilized onto the working electrode 12 and the reference electrode 13 along the sample path 18 in the width direction.

In the first embodiment of the present invention, the biosensor 10 further includes. sensing electrodes 21 and 23 formed on the inner surfaces of the upper insulating substrate 17 and the lower insulating substrate 11 in the longitudinal direction around a sample injection port 18a, and a sensing electrode 22 formed on the inner surface of the lower insulating substrate 11 at the opposite side of the sample injection port 18a.

Accordingly, one sensing electrode 23 is formed on the upper insulating substrate 17, and four electrodes including two sensing electrodes 21 and 22, the working electrode 12, and the reference electrode 13 are formed on the lower insulating substrate 11. The two sensing electrodes 21 and 22 formed on the lower insulating substrate 11 are disposed at the outside of the working electrode 12 and the reference electrode 13, i.e., around the sample injection port 18a and at the opposite side of the sample injection port 18a, at a predetermined distance.

Like the working electrode 12 and the reference electrode 13, the sensing electrodes 21, 22, and 23 extend from the sample path 18, through which a sample passes, to a connection terminal of the biosensor 10 to be connected to a socket of a measuring device (not shown), and are disposed in front and rear of the working electrode 12 and the reference electrode 13 with respect to the flow direction of the sample in the sample path 18.

For a better understanding of the present invention, the sensing electrode formed on the upper insulating substrate 17 will be referred to as an upper sensing electrode, and the sensing electrode formed on the lower insulating substrate 11 will be referred to as a lower sensing electrode. Moreover, the sensing electrode formed on the lower insulating substrate 11 around the sample injection port 18a, i.e., at the outside of the working electrode 12 (adjacent to the sample injection port 18a) will be referred to as a first lower sensing electrode, and the sensing electrode formed on the lower insulating substrate 11 at the opposite side of the sample injection port 18a, i.e., at the outside of the reference electrode 13 (far away from the sample injection port 18a), will be referred to as a second lower sensing electrode.

In the first embodiment, the upper sensing electrode 23 and the first lower sensing electrode 21 are used to determine whether a sample is injected. When the sample is injected through the sample injection port 18a and comes in contact with the upper sensing electrode 23 and the first lower sensing electrode 21 around the sample injection port 18a, the time point at which the upper sensing electrode 23 and the first lower sensing electrode 21 are electrically connected to each other by the sample is detected.

The second lower sensing electrode 22 is used to determine whether the sample is completely filled in the sample path 18. The time point at which the sample sequentially passes through the first lower sensing electrode 21, the working electrode 12, and the reference electrode 13 and reaches the second lower sensing electrode 22 will be detected as a time point at which the sample is completely filled. This time point will be detected when the first lower sensing electrode 21 and the second lower sensing electrode 22 are electrically connected to each other by the sample.

As a result, in the first embodiment, it is possible to determine whether or not the sample is completely filled after it is injected and determine the time point at which the sample is initially injected and the time point at which the sample is completely filled, and thus it is possible to measure the time until the sample is completely filled after it is injected. That is, the measuring device into which the biosensor 10 is inserted can measure the flow characteristics of the sample and the flow rate by detecting the conductive state of the respective electrodes.

The sensing electrodes provided by the present invention in addition to the working electrode and the reference electrode may be formed of the same material and by the same method as the existing electrodes. For example, it is possible to form the sensing electrodes by a known electrode formation process, that is, by a thin film formation process such as etching, screen printing, or sputtering.

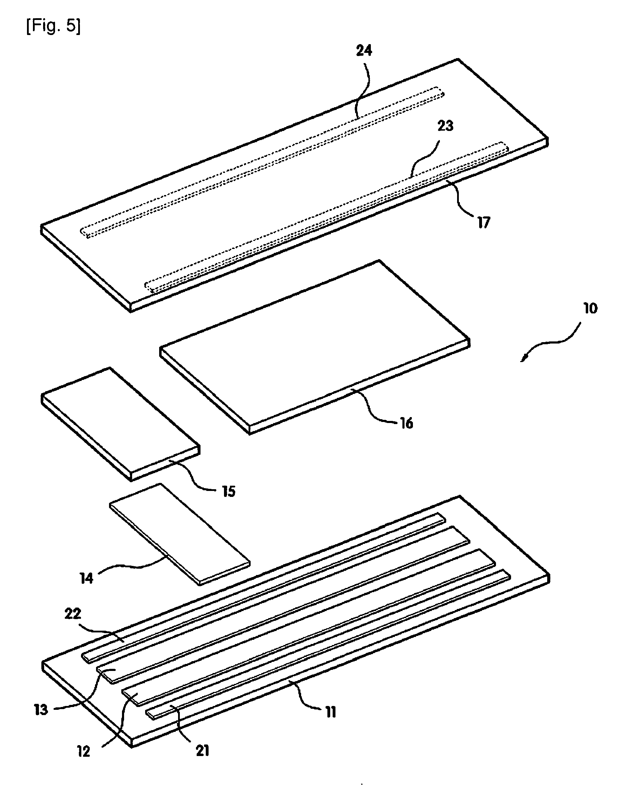

Next, FIG. 5 is an exploded perspective view showing a biosensor in accordance with a second embodiment of the present invention. In the same manner as the first embodiment, four electrodes are formed on the inner surface of the lower insulating substrate 11 by adding two lower sensing electrodes 21 and 22. Moreover, two upper sensing electrodes 23 and 24 are formed on the inner surface of the upper insulating substrate 17 at positions corresponding to the lower sensing electrodes 21 and 22. Compared to the first embodiment, the first lower sensing electrode 21 and the second lower sensing electrode 22 formed on the lower insulating substrate 11, and the upper sensing electrode 23 (hereinafter referred to as a first upper sensing electrode) formed on the upper insulating substrate 17 around the sample injection port 18a are the same as those of the first embodiment; however, the upper sensing electrode 23 (hereinafter referred to as a second upper sensing electrode) is further provided on the upper insulating substrate 17 at a position corresponding to the second lower sensing electrode 22, i.e., at the opposite side of the sample injection port 18a.

In the above-described second embodiment, the first upper sensing electrode 23 and the first lower sensing electrode 21 are used to determine whether the sample is injected. When the sample is injected through the sample injection port 18a and comes in contact with the first upper sensing electrode 23 and the first lower sensing electrode 21 formed around the sample injection port 18a, the time point at which the first upper sensing electrode 23 and the first lower sensing electrode 21 are electrically connected to each other by the sample is detected.

The second upper sensing electrode 24 and the second lower sensing electrode 22 are used to determine whether the sample is completely filled in the sample path 18 as it reaches the reference electrode 13. The time point at which the sample sequentially passes through the first lower sensing electrode 21, the working electrode 12, and the reference electrode 13 and reaches the second upper sensing electrode 24 and the second lower sensing electrode 22 will be detected as a time point at which the sample is completely filled. This time point will be detected when the second upper sensing electrode 24 and the second lower sensing electrode 22 are electrically connected to each other by the sample.

As a result, in the second embodiment, like the first embodiment, it is possible to determine whether or not the sample is completely filled after it is injected and determine the time point at which the sample is initially injected and the time point at which the sample is completely filled, and thus it is possible to measure the time until the sample is completely filled after it is injected. That is, the measuring device into which the biosensor 10 is inserted can measure the flow characteristics of the sample and the flow rate by detecting the conductive state of the respective electrodes.

As such, in the first and second embodiments of the present invention, it is possible to accurately measure the time until the sample is completely filled by means of the electrodes, and the measured time can be used as a means for obtaining a correction coefficient used to correct the error in the measured value according to a difference in reactivity.

In more detail, since the flow rate of the sample may differ according to the flow characteristics of the sample, it is possible to estimate the properties of the sample from the difference in flow rate. Moreover, the flow characteristics of the sample may affect the measurement result and cause an error. Accordingly, if the difference in reactivity according to the properties of the sample is measured numerically by a test and stored in the measuring device, it is possible to correct the difference in reactivity by measuring the flow rate.

For example, in the case where blood is used as the sample, if the number of red blood cells is low, the solubility of the reagent (enzyme reaction layer) is increased to increase the reactivity, and thus the measured value is increased. On the contrary, if the number of red blood cells is high, the solubility is reduced to decrease the reactivity. Moreover, since the number of red blood cells is in inverse proportion to the flow rate of blood, if the measuring device calculates the number of red blood cells by measuring the flow rate of blood, it is possible to correct the difference in reactivity.

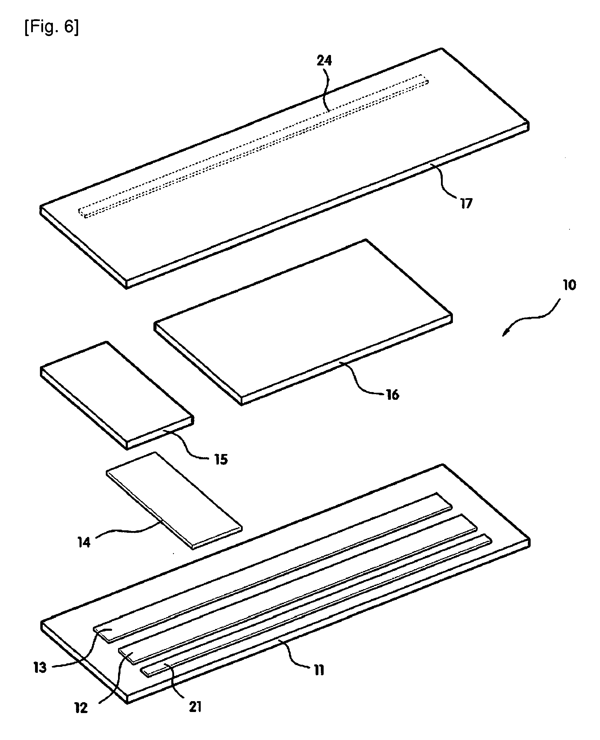

Meanwhile, FIG. 6 is an exploded perspective view showing a biosensor in accordance with a third embodiment of the present invention. In this embodiment, three electrodes are formed on the lower insulating substrate 11 by adding a lower sensing electrode 21 around the sample injection port 18a, i.e., at the outside of the working electrode 12, in addition to the working electrode 12 and the reference electrode 13. Moreover, an upper sensing electrode 24 is formed on the upper insulating substrate 17 at the opposite side of the sample injection port 18a (at a side far away from the sample injection port 18a). The upper sensing electrode 24 on the upper insulating substrate 17 is positioned at the outside of the reference electrode 13 at a predetermined distance when the upper and lower insulating substrates 11 and 17 are bonded.

In the above-described third embodiment, the lower sensing electrode 21 and the upper sensing electrode 24 are used to determine whether the sample is completely filled in the sample path 18 as it reaches the reference electrode 13. The time point at which the sample sequentially passes through the lower sensing electrode 21, the working electrode 12, and the reference electrode 13 and reaches the upper sensing electrode 24 will be detected as a time point at which the sample is completely filled. This time point will be detected when the lower sensing electrode 21 and the upper sensing electrode 24 are electrically connected to each other by the sample.

As a result, the measuring device into which the biosensor 10 is inserted can determine the time point at which the sample is completely filled by detecting the conductive state of the lower sensing electrode 21 and the upper sensing electrode 24.

Moreover, in the third embodiment, it is also possible to estimate the flow characteristics of the sample and the flow rate using the lower sensing electrode 21 and the working electrode 12. That is, since the measuring device can detect the time point at which the two electrodes 12 and 21 are electrically connected to each other by the sample, which first comes in contact with the lower sensing electrode 21 and then comes in contact with the working electrode 12, it is possible to estimate the flow rate of the sample by measuring the time until the sample reaches the upper sensing electrode 24 from that time point. As a result, the estimated flow rate of the sample can be used as a means for obtaining a correction coefficient used to correct the error in the measured value according to a difference in reactivity.

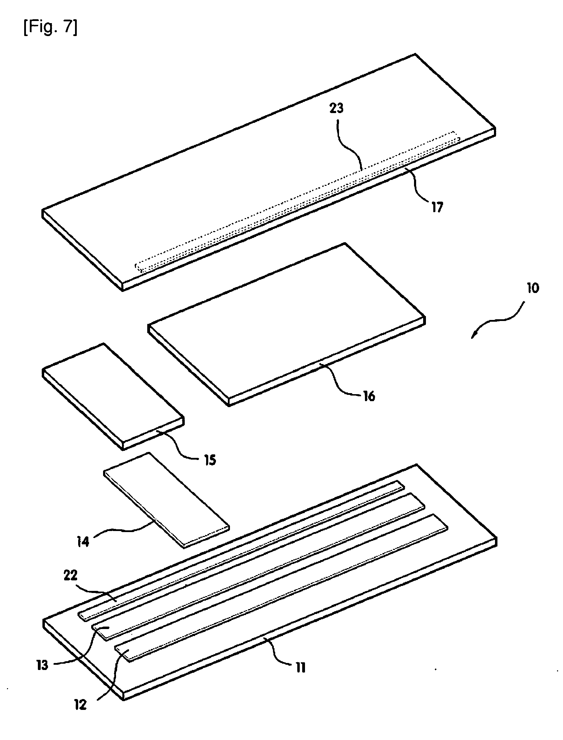

Finally, FIG. 7 is an exploded perspective view showing a biosensor in accordance with a fourth embodiment of the present invention. In this embodiment, three electrodes are formed on the lower insulating substrate 11 by adding a lower sensing electrode 22 at the opposite side of the sample injection port 18a, i.e., at the outside of the reference electrode 13(at a side far away from the sample injection port 18a), in addition to the working electrode 12 and the reference electrode 13. Moreover, an upper sensing electrode 23 is formed on the upper insulating substrate 17 around the sample injection port 18a. The upper sensing electrode 23 on the upper insulating substrate 17 is positioned at the outside of the working electrode 12 at a predetermined distance when the upper and lower insulating substrates 11 and 17 are bonded.

In the above-described fourth embodiment, the lower sensing electrode 22 and the upper sensing electrode 23 are used to determine whether the sample is completely filled in the sample path 18 as it reaches the reference electrode 13. The time point at which the sample sequentially passes through the upper sensing electrode 23, the working electrode 12, and the reference electrode 13 and reaches the lower sensing electrode 22 will be detected as a time point at which the sample is completely filled. This time point will be detected when the upper sensing electrode 23 and the lower sensing electrode 22 are electrically connected to each other by the sample.

As a result, the measuring device into which the biosensor 10 is inserted can determine the time point at which the sample is completely filled by detecting the conductive state of the lower sensing electrode 22 and the upper sensing electrode 23.

Moreover, in the fourth embodiment, it is also possible to estimate the flow characteristics of the sample and the flow rate using the upper sensing electrode 23 and the working electrode 12. That is, since the measuring device can detect the time point at which the two electrodes 12 and 23 are electrically connected to each other by the sample, which first comes in contact with the upper sensing electrode 23 and then comes in contact with the working electrode 12, it is possible to estimate the flow rate of the sample by measuring the time until the sample reaches the lower sensing electrode 22 from that time point. As a result, the estimated flow rate of the sample can be used as a means for obtaining a correction coefficient used to correct the error in the measured value according to a difference in reactivity.

As described above, according to the biosensor of the present invention, in which the sensing electrodes are formed on the upper insulating substrate and the lower insulating substrate such that, using the sensing electrodes, the measuring device can determine whether a sample (e.g., blood) is completely filled in the sample path when the sample is injected through the sample injection port. Moreover, since the measuring device can determine a time point at which the sample is injected and a time point at which the sample is completely filled by means of the electrodes provided in the biosensor, it is possible to estimate the flow characteristics of the sample and the flow rate and use the estimated values to correct the measurement result, thus obtaining a more accurate measurement result.

The invention has been described in detail with reference to preferred embodiments thereof. However the scope of protection is defined in the appended claims.

## Claims

1. A biosensor, comprising:
an upper insulating substrate 17;
a lower insulating substrate 11;
a working electrode 12;
a reference electrode 13, the working electrode 12 and the reference electrode 13 being formed on the inner surface of the lower insulating substrate 11;
spacers 15 and 16 interposed between the upper insulating substrate 17 and the lower insulating substrate 11, the spacers and the insulating substrates being configured to form a sample path 18 via which sample flows;
an enzyme reaction layer 14 as a reagent is immobilized onto the working electrode 12 and the reference electrode 13 along the sample path 18 in the width direction; and at least one sensing electrode formed on the upper insulating substrate 17 and the lower insulating substrate 11, respectively, the sensing electrodes being disposed adjacent to a sample injection port 18a and at the opposite side of the sample injection port 18a, respectively, such that the working electrode 12 and the reference electrode 13 are interposed therebetween, the sensing electrodes extending substantially from the sample path 18 to a connection terminal to be connected to a socket of a measuring device.

2. The biosensor of claim 1, wherein sensing electrodes 21 and 23 are formed on the inner surface of the upper insulating substrate 17 and the inner surface of the lower insulating substrate 11 at positions opposite to each other around the sample injection port 18a, respectively, and a sensing electrode 22 is formed on the inner surface of the lower insulating substrate 11 at the opposite side of the sample injection port 18a.

3. The biosensor of claim 1, wherein sensing electrodes 21, 22, 23, and 24 are formed on the inner surface of the upper insulating substrate 17 and the inner surface of the lower insulating substrate 11 at positions opposite to each other around the sample injection port 18a and at the opposite side of the sample injection pout 18a, respectively.

4. The biosensor of claim 1, wherein a sensing electrode 24 is formed on the inner surface of the upper insulating substrate 17 at the opposite side of the sample injection port 18a, and a sensing electrode 21 is formed on the inner surface of the lower insulating substrate 11 around the sample injection port 18a.

5. The biosensor of claim 1, wherein a sensing electrode 23 is formed on the inner surface of the upper insulating substrate 17 around the sample injection port 18a, and a sensing electrode 22 is formed on the inner surface of the lower insulating substrate 11 at the opposite side of the sample injection port 18a.

## Patentansprüche

1. Biosensor mit:
einem oberen isolierenden Substrat 17;
einem unteren isolierenden Substrat 11;
einer Arbeitselektrode 12;
einer Referenzelektrode 13, wobei die Arbeitselektrode 12 und die Referenzelektrode 13 auf der Innenseite des unteren isolierenden Substrats 11 ausgebildet sind;
Abstandhaltern 15 und 16, die zwischen dem oberen isolierenden Substrat 17 und dem unteren isolierenden Substrat 11 angeordnet sind, wobei die Abstandhalter und die isolierenden Substrate derart ausgebildet sind, dass sie einen Probenweg 18 bilden, entlang welchem eine Probe fließt;
wobei eine Enzymreaktionsschicht 14 als Reagenz in Breitenrichtung entlang dem Probenweg 18 auf der Arbeitselektrode 12 und der Referenzelektrode 13 immobilisiert ist; und
mindestens einer Sensorelektrode, die jeweils auf dem oberen isolierenden Substrat 17 und dem unteren isolierenden Substrat 11 ausgebildet ist, wobei die Sensorelektroden jeweils nahe einem Probeninjektionsport 18a und auf der dem Probeninjektionsport 18a entgegengesetzten Seite angeordnet sind, so dass die Arbeitselektrode 12 und die Referenzelektrode 13 zwischen diesen angeordnet sind, wobei die Sensorelektroden sich im Wesentlichen von dem Probenweg 18 zu einer mit einer Anschlussbuchse einer Messvorrichtung verbindbaren Anschlussklemme erstrecken.

2. Biosensor nach Anspruch 1, bei welchem Sensorelektroden 21 und 23 auf der Innenseite des oberen isolierenden Substrats 17 und der Innenseite des unteren isolierenden Substrats 11 jeweils an einander gegenüberliegenden Positionen um den Probeninjektionsport 18a ausgebildet sind und eine Sensorelektrode 22 auf der Innenseite des unteren isolierenden Substrats 11 auf der dem Probeninjektionsport 18a entgegengesetzten Seite ausgebildet ist.

3. Biosensor nach Anspruch 1, bei welchem Sensorelektroden 21, 22, 23 und 24 auf der Innenseite des oberen isolierenden Substrats 17 und der Innenseite des unteren isolierenden Substrats 11 jeweils an einander gegenüberliegenden Positionen um den Probeninjektionsport 18a und auf der dem Probeninjektionsport 18a entgegengesetzten Seite ausgebildet sind.

4. Biosensor nach Anspruch 1, bei welchem eine Sensorelektrode 24 auf der Innenseite des oberen isolierenden Substrats 17 auf der dem Probeninjektionsport 18a entgegengesetzten Seite ausgebildet ist, und eine Sensorelektrode 21 auf der Innenseite des unteren isolierenden Substrats 11 um den Probeninjektionsport 18a herum ausgebildet ist.

5. Biosensor nach Anspruch 1, bei welchem eine Sensorelektrode 23 auf der Innenseite des oberen isolierenden Substrats 17 um den Probeninjektionsport 18a herum ausgebildet ist und eine Sensorelektrode 22 auf der Innenseite des unteren isolierenden Substrats 11 auf der dem Probeninjektionsport 18a entgegengesetzten Seite ausgebildet ist.

## Revendications

1. Biocapteur, comprenant :
un substrat isolant supérieur 17 ;
un substrat isolant inférieur 11 ;
une électrode de travail 12 ;
une électrode de référence 13, l'électrode de travail 12 et l'électrode de référence 13 étant formées sur la surface intérieur du substrat isolant inférieur 11 ;
des entretoises 15 et 16 étant intercalées entre le substrat isolant supérieur 17 et le substrat isolant inférieur 11, les entretoises et les substrats isolants étant configurés de façon à former un chemin de passage pour l'échantillon 18, par lequel s'écoule l'échantillon ;
une couche de réaction enzymatique 14 en tant que réactif est immobilisée sur l'électrode de travail 12 et l'électrode de référence 13 le long du chemin de passage pour l'échantillon 18 dans la direction latérale ; et au moins une électrode de captage formée respectivement sur le substrat isolant supérieur 17 et le substrat isolant inférieur 11, les électrodes de captage étant disposées respectivement contre un orifice d'injection d'échantillon 18a et sur le côté opposé de l'orifice d'injection d'échantillon 18a, de telle sorte que l'électrode de travail 12 et l'électrode de référence 13 soient incorporées entre eux, les électrodes de captage s'étendant sensiblement du chemin de passage pour l'échantillon 18 à une borne de connexion devant être connectée à une prise prévue sur le dispositif de mesure.

2. Biocapteur selon la revendication 1, dans lequel les électrodes de captage 21 et 23 sont formées respectivement sur la surface intérieure du substrat isolant supérieur 17 et sur la surface intérieure du substrat isolant inférieur 11 sur des positions opposées l'une à l'autre autour de l'orifice d'injection d'échantillon 18a, et une électrode de captage 22 est formée sur la surface intérieure du substrat isolant inférieur 11 sur le côté opposé de l'orifice d'injection d'échantillon 18a.

3. Biocapteur selon la revendication 1, dans lequel les électrodes de captage 21, 22, 23 et 24 sont formées respectivement sur la surface intérieure du substrat isolant supérieur 17 et sur la surface intérieure du substrat isolant inférieur 11 sur des positions opposées l'une à l'autre autour de l'orifice d'injection d'échantillon 18a et sur le côté opposé de l'orifice d'injection d'échantillon 18a.

4. Biocapteur selon la revendication 1, dans lequel une électrode de captage 24 est formée sur la surface intérieure du substrat isolant supérieur 17 sur le côté opposé de l'orifice d'injection d'échantillon 18a, et une électrode de captage 21 est formée sur la surface intérieure du substrat isolant inférieur 11 autour de l'orifice d'injection d'échantillon 18a.

5. Biocapteur selon la revendication 1, dans lequel une électrode de captage 23 est formée sur la surface intérieure du substrat isolant supérieur 17 autour de l'orifice d'injection d'échantillon 18a, et une électrode de captage 22 est formée sur la surface intérieure du substrat isolant inférieur 11 sur le côté opposé de l'orifice d'injection d'échantillon 18a.
